(19) Europäisches Patentamt
European Patent Office
Office européen des brevets

(11) Veröffentlichungsnummer: **0 093 330**
**B1**

(12) # EUROPÄISCHE PATENTSCHRIFT

(45) Veröffentlichungstag der Patentschrift: **16.07.86**

(51) Int. Cl.⁴: **G 03 G 5/06,** C 07 D 249/20

(21) Anmeldenummer: **83103861.7**

(22) Anmeldetag: **20.04.83**

(54) **Elektrographische Aufzeichnungsmaterialien mit speziellen Ladungsträger transportierenden Verbindungen.**

(30) Priorität: **29.04.82 DE 3215968**

(43) Veröffentlichungstag der Anmeldung: **09.11.83 Patentblatt 83/45**

(45) Bekanntmachung des Hinweises auf die Patenterteilung: **16.07.86 Patentblatt 86/29**

(84) Benannte Vertragsstaaten: **BE CH DE FR GB IT LI NL**

(56) Entgegenhaltungen:
**DE - A - 2 346 803**
**FR - A - 1 238 483**
**FR - A - 1 493 173**
**FR - A - 2 383 464**
**US - A - 2 501 188**

(73) Patentinhaber: **BASF Aktiengesellschaft, Carl-Bosch-Strasse 38, D-6700 Ludwigshafen (DE)**

(72) Erfinder: **Eilingsfeld, Heinz, Dr., Pierstrasse 9 A, D-6710 Frankenthal (DE)**
Erfinder: **Etzbach, Karl-Heinz, Dr., Bensheimer Ring 9A, D-6710 Frankenthal (DE)**
Erfinder: **Hoffmann, Gerhard, Dr., Pappelstrasse 22, D-6701 Otterstadt (DE)**
Erfinder: **Neumann, Peter, Dr., Franz-Schubert-Strasse 1, D-6908 Wiesloch (DE)**

**EP 0 093 330 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft elektrophotographische Aufzeichnungsmaterialien mit elektrisch leitenden Trägern, Ladungsträger erzeugenden Verbindungen bzw. Sensibilisatoren und speziellen Ladungsträger transportierenden Verbindungen.

Elektrophotographische Verfahren, dafür benötigte Materialien und verschiedene Varianten für den Aufbau von Aufzeichnungsmaterialien sind bekannt. Vorteilhaft für den Einsatz im Reproduktionssektor sind Materialien aus polymeren Bindemitteln, die an spezielle Anforderungen des jeweiligen Einsatzgebietes angepasst werden können, niedermolekularen organischen Verbindungen, die in den Bindemitteln auch in höheren Konzentrationen löslich und zu einem Transport von Ladungsträgern des elektrischen Stromes befähigt sind, sowie Verbindungen, insbesondere Farbstoffe oder Pigmente, die durch Absorption des bildmässig eingestrahlten, aktinischen Lichts Ladungsträger des elektrischen Stromes erzeugen und diese unter Mithilfe des von aussen durch die elektrostatische Oberflächenladung aufgeprägten elektrischen Feldes auf die Ladung transportierenden Verbindungen übertragen können. Diese Ladungsträger erzeugenden Verbindungen können je nach Einsatzgebiet des Aufzeichnungsmaterials als eigene Schicht innerhalb einer Kompositstruktur eingebracht werden (vgl. DE-OS 22 20 408) oder in Form monodispers gelöster Farbstoffmoleküle in der Mischung aus Bindemittel und Ladungsträger transportierende Verbindungen vorhanden sein (vgl. DE-PS 1 058 836). Das in der DE-OS 22 20 408 beschriebene mehrlagige elektrophotographische Aufzeichnungsmaterial besteht aus einem elektrisch leitfähigen Trägermaterial, einer ersten, Farbstoff enthaltenden, etwa 0,005 bis 2 µm dikken, durch Belichtung mit aktinischem Licht Ladungsträger des elektrischen Stromes erzeugenden Schicht aus im Dunkeln isolierenden, organischen Materialien mit mindestens einer Ladungen transportierenden Verbindung.

Es ist auch bekannt, photohalbleitende organische Verbindungen zur Herstellung von elektrophotographischen Druckformen und insbesondere elektrophotographischen Offsetdruckformen zu verwenden (vgl. DE-PS 1 117 391 und 1 120 875, DE-AS 15 22 497 und 27 26 116).

Die gestiegenen Anforderungen an Reproduktionssysteme verlangen eine Vielfalt von Aufzeichnungsmaterialien und -systemen, um für spezielle Probleme optimale Lösungen aussuchen zu können. Gewünscht ist eine hohe Lichtempfindlichkeit, eine gute Auflösung und gute Betonerung. Die oft beanstandete ungenügende Betonerung, die auf eine ungünstige Feldstärkedifferenzierung zwischen belichteten und unbelichteten Flächen hinweist, ist hierbei oft auf eine zu hohe Dunkelleitfähigkeit des Aufzeichnungsmaterials im beladenen Zustand zurückzuführen, so dass eine ungenügende Oberflächenladungsdichte vor der aktinischen bildmässigen Belichtung vorliegt.

Der vorliegenden Erfindung lag die Aufgabe zugrunde, weitere elektrophotographische Aufzeichnungsmaterialien insbesondere für die Herstellung von elektrophotographischen Druckformen wie Offsetdruckformen zu entwickeln, die bei hoher Lichtempfindlichkeit, guter Auflösung und Verarbeitung gleichzeitig ein geringes Dunkelleitvermögen aufweisen.

Es wurde nun gefunden, dass man so verbesserte elektrophotographische Aufzeichnungsmaterialien mit elektrisch leitenden Trägern, Ladungsträger erzeugenden Verbindungen und Ladungsträger transportierenden Verbindungen erhält, wenn sie als Ladungsträger transportierende Verbindungen solche der Formel (I) enthalten

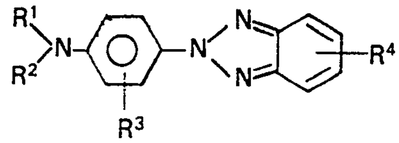 (I)

worin bedeuten:

$R^1$ und $R^2$ = Wasserstoff, Alkyl, Allyl, Benzyl oder ggf. subst. Phenyl, gleich oder verschieden, oder

$R^1$ und $R^2$ gemeinsam

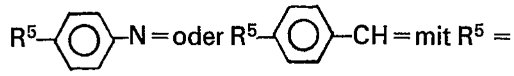
oder, mit $R^5$ = $NR^1R^2$

$R^3$ = Wasserstoff, Alkyl, Alkoxy oder Halogen,

$R^4$ = Wasserstoff, Alkyl, Alkoxy, Vinyl, Allyl, Dialkylamino, Nitro, Cyan oder Acryloyl.

Bevorzugt sind hierbei die Verbindungen, bei denen in Formel (I)

$R^1$ = Wasserstoff, Alkyl, Benzyl oder Phenyl

$R^2$ = Alkyl, Benzyl oder Phenyl

$R^3$ = Wasserstoff und

$R^4$ = Wasserstoff, Alkoxy oder Halogen bedeuten.

Bevorzugt ist der $R^4$-Rest in 6-Stellung des Benztriazols-1,2,3.

Beispiele sehr geeigneter Verbindungen sind:

(1) 2-(4'-Diethylaminophenyl)- benztriazol-1,2,3

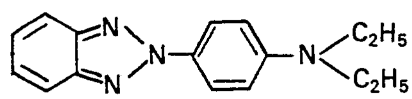

$C_{16}H_{18}N_4$
Schmp. 101–102 °C

(2) 2-(4'-Diethylaminophenyl) -6-methoxybenztriazol-1,2,3

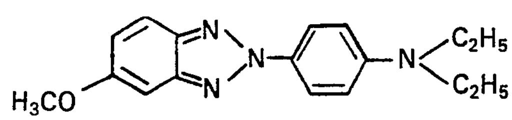

$C_{17}H_{20}N_4O$
Schmp. 95–96 °C

(3) 2-(4'-Diethylaminophenyl) -6-chlor-benztriazol -1,2,3

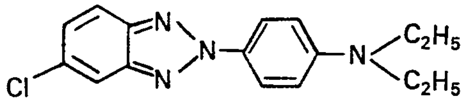

$C_{16}H_{17}N_4Cl$
Schmp. 101–102 °C

(4) 2-(4'-Ethylbenzylaminophenyl) -benztriazol-1,2,3

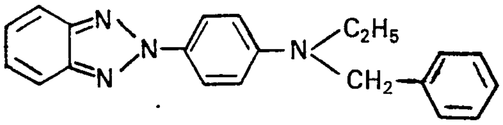

$C_{21}H_{20}N_4$
Schmp. 120–122 °C

(5) 2-(4'-Ethylbenzylaminophenyl) -6-methoxy-benztriazol-1,2,3

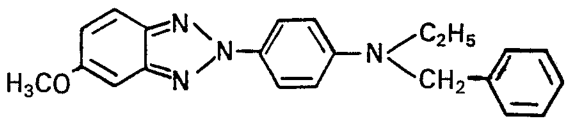

$C_{22}H_{22}N_4O$
Schmp. 105–106 °C

(6) 2-(2'-Methyl -4'- diethylaminophenyl)-benztriazol-1,2,3

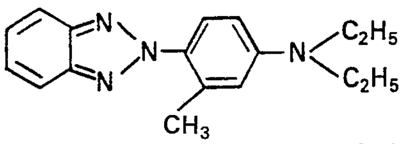

$C_{17}H_{20}N_4$
Schmp. 77–79 °C

(7) 2-(4'-Dibenzylaminophenyl) -6-methoxy-benztriazol-1,2,3

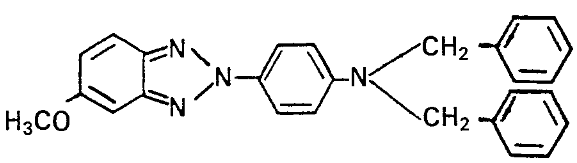

$C_{27}H_{24}N_4O$
Schmp. 152–154 °C

(8) 2-(4'-Diethylaminophenyl) -6-methylbenztriazol-1,2,3

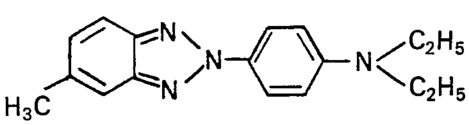

$C_{17}H_{20}N_4$
Schmp. 102–103 °C

(9) 2-(4'-Methylphenylaminophenyl) -benztriazol-1,2,3

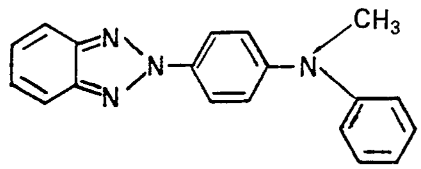

$C_{19}H_{16}N_4$
Schmp. 115–117 °C

(10) 2-(4'-Diethylaminophenyl) -6-ethoxy-benztriazol-1,2,3

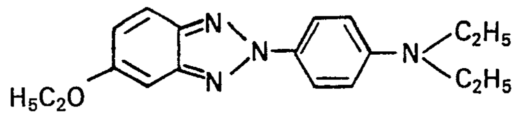

$C_{18}H_{22}N_4O$
Schmp. 107–108 °C

(11) 2-(4'-Diethylaminophenyl) -6-benzyloxy-benztriazol-1,2,3

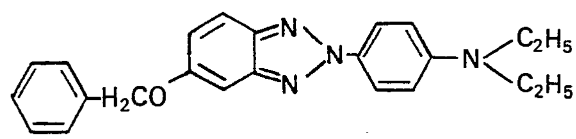

$C_{23}H_{24}N_4O$
Schmp. 113–115 °C

(12) 2-(4'-Methylphenylaminophenyl) -6-methoxy-benztriazol-1,2,3

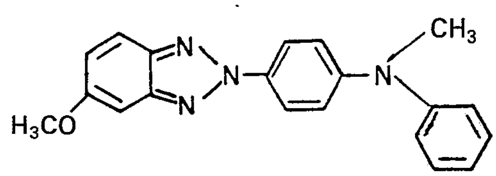

$C_{20}H_{18}N_4O$
Schmp. 109–110 °C

(13) 2-(4'-Ethylbenzylaminophenyl) -6-ethoxy-benztriazol-1,2,3

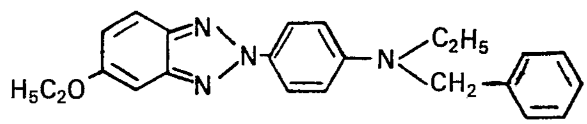

$C_{23}H_{24}N_4$
Schmp. 91–92 °C

(14) 2-(4'-Diethylaminophenyl) -6-diethylamino-benztriazol-1,2,3

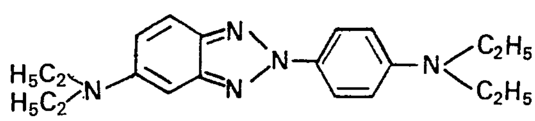

$C_{20}H_{27}N_5$
Schmp. 95–96 °C

(15) 2-(4'-Diethylaminophenyl) -6-fluorbenztriazol-1,2,3

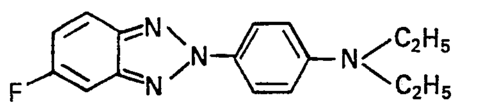

$C_{16}H_{17}N_3F$
Schmp. 94–95 °C

Die erfindungsgemäss verwendeten substituierten Phenylbenztriazole sind nach bekannten Methoden der organischen Chemie darstellbar. Sie werden beispielsweise erhalten aus ortho-Aminoazoverbindungen der Formel

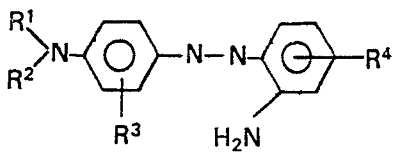

(II)

oder ortho-Nitroazoverbindungen der Formel

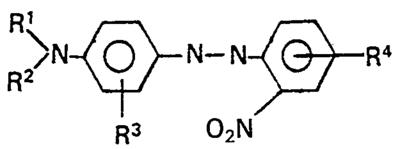

(III),

in denen $R^1$, $R^2$, $R^3$ und $R^4$ die oben genannten Bedeutungen haben, nach den in Houben-Weyl, Methoden der organischen Chemie, 4. Auflage, Band X/3, S. 425ff, beschriebenen Methoden. Die Verbindungen der Formeln II und III sind bekannt oder können nach Standardverfahren hergestellt werden.

Die erfindungsgemäss verwendeten bei Belichtung Ladungsträger transportierenden Verbindungen können mit Vorteil sowohl in einschichtig als auch in mehrschichtig auf elektroleitfähige Träger aufgebrachten Aufzeichnungssystemen verwendet werden.

Geeignete einschichtige Systeme weisen bevorzugt auf einem leitfähigen Trägermaterial eine Schicht aus (a) 45 bis 75 Gewichtsteilen eines Bindemittels, (b) 30 bis 60, insbesondere 35 bis 50 Gewichtsteilen einer der erfindungsgemäss verwendeten, Ladungsträger transportierenden Verbindungen, (c) ggf. 5 bis 25 Gewichtsteilen eines weiteren, im wesentlichen inaktiven Bindemittels und (d) 0,05 bis 0,8 Gewichtsteilen einer bei aktinischer Belichtung Ladungsträger erzeugenden Verbindung, insbesondere eines geeigneten Farbstoffs auf. Die Schichten werden mit Vorteil aus einer ca. 5 gew.-%igen Lösung in einem geeigneten organischen Lösungsmittel auf das gereinigte leitfähige Trägermaterial so aufgebracht, dass nach dem Abflüften des Lösungsmittels eine Trockenschichtdicke von ca. 0,8 bis 40 µm (je nach Verwendungszweck, bei elektrophotographischen Druckformen insbesondere 0,8 bis 6 µm) resultiert.

Geeignete Mehrschichtsysteme nach Anspruch 3 haben auf einem elektroleitfähigen Trägermaterial z.B. (a) eine Ladungsträger erzeugende Schicht und (b) eine Ladungstransportschicht aus (b1) 30 bis 60 Gewichtsteilen mindestens einer Ladungsträger transportierenden Verbindungen der Formel (I), (b2) 45 bis 75 Gewichtsteilen eines organischen Bindemittels und (b3) ggf. 5 bis 25 Gewichtsteilen weiterer, die mechanischen Eigenschaften der Schicht verbessernden Zusätze. Die erste Schicht wird vorteilhaft in einer Dickè von 0,005 bis 5, insbesondere 0,1 bis 0,9 µm aus Lösung in einem geeigneten Lösungsmittel auf das Trägermaterial aufgetragen. Nach dem Auftrag erfolgt der Auftrag der zweiten Schicht in einer Dicke, dass nach dem Trocknen der Kompositstruktur eine Schichtdicke von 5 bis 25, insbesondere 7 bis 15 µm resultiert.

Als elektrisch leitende Träger sind prinzipiell alle leitfähigen Trägermaterialien verwendbar, soweit sie für das Einsatzgebiet geeignet sind. Bevorzugt sind je nach Einsatzgebiet der Aufzeichnungsmaterialien Aluminium-, Zink-, Magnesium-, Kupfer- oder Mehrmetallplatten, z.B. rohe oder vorbehandelte, z.B. aufgerauhte und/oder anodisierte Aluminiumbleche, Aluminiumfolien, Polymerfilme mit metallisierter Oberfläche wie aluminiumbedampfte Polyethylenterephthalatfilme oder auch elektrisch leitende Spezialpapiere. Träger für Druckformen haben vorteilhaft eine Dicke von 0,08 bis ca. 0,3 mm.

Die Art der geeigneten organischen Bindemittel für die Schichten richtet sich nach dem beabsichtigten Verwendungszweck der Aufzeichnungsmaterialien. Für den Kopiersektor eignen sich z.B. Celluloseether, Polyesterharze, Polyvinylchloride, Polycarbonate, Copolymere, wie Styrol-Maleinsäureanhydrid-Copolymere oder Vinylchlorid-Maleinsäureanhydrid-Copolymere oder Mischungen solcher Bindemittel. Bei ihrer Auswahl spielen ihre filmbildenden und elektrischen Eigenschaften, ihre Haftfestigkeit auf dem Trägermaterial und ihre Löslichkeitseigenschaften eine besondere Rolle. Insbesondere bei Aufzeichnungsmaterialien für die Herstellung elektrophotographischer Druckplatten und besonders bei denen für den Offsetdruck sind solche besonders geeignet, die in basischen wässrigen oder alkoholischen Lösungsmitteln löslich sind. Dies sind vor allem Substanzen mit alkalilöslich machenden Gruppen wie Anhydrid-, Carboxyl-, Sulfonsäure-, Phenol-oder Sulfonimid-Gruppierungen. Bevorzugt sind Bindemittel, insbesondere solche mit hohen Säurezahlen, die in basischen wässrig-alkoholischen Lösungsmittelsystemen leicht löslich sind und ein mittleres Molekulargewicht (Gewichtsmittel) von 800 bis 50 000 und insbesondere 1 500 bis 10 000 aufweisen. Geeignet sind z.B. Copolymerisate aus Methacrylsäure und Methacrylsäureestern, besonders Copolymerisate aus Styrol und Maleinsäureanhydrid und aus Styrol, Methacrylsäure und Methacrylsäureester, soweit sie die vorstehende Löslichkeitsbedingung aufweisen. Obwohl bekanntermassen Bindemittel mit freien Carboxylgruppen die Dunkelleitfähigkeit der elektrophotographischen Schichten in unerwünschter Weise erhöhen und dadurch zu schlechten Betonerungsergebnissen führen, lassen sich solche Bindemittel leicht an die erfindungsgemäss verwendeten Benztriazole anpassen. So hat sich gezeigt, dass Copolymerisate aus Styrol, Maleinsäureanhydrid und Acryl- oder Methacrylsäure, die einen Anteil von einpolymerisiertem Maleinsäureanhydrid von 5 bis 50 Gew.-% und einen Anteil von einpolymerisierter Acryl- oder Methacrylsäure von 5 bis zu 35 und insbesondere 10 bis 30 Gew.-% aufweisen, befriedigende elektrophotographische Schichten mit hinreichender Dunkelleitfähigkeit ergeben. Sie weisen eine hervorragende Löslichkeit in Auswaschmitteln aus 75 Gew.-% Wasser, 23 Gew.-% Isobutanol und 2 Gew.-% Soda auf, sind aber in offsettypischem Wischwasser unlöslich.

Geeignete Ladungsträger erzeugende Verbindungen bzw. Sensibilisatoren sind z.B. für einschichtig aufgetragene Systeme, wie sie auch zur Herstellung elektrophotoraphischer Druckformen dienen, Farbstoffe aus der Triarylmethanreihe, Xanthenfarbstoffe und Cyaninfarbstoffe. Sehr gute Ergebnisse wurden mit den erfindungsgemäss verwendeten Verbindungen der Formel I mit Rhodamin B (C.I. 45170), Rhodamin 6 G (C.I. 45160), Malachitgrün (C.I. Basic Green 4; C.I. 42000), Methylviolett (C.I. 42535) oder Kristallviolett (C.I. 42555) erhalten. Bei mehrschichtig aufgetragenen Systemen liegt der Farbstoff oder das Pigment in einer separaten Ladungsträger erzeugenden Schicht vor. Hier sind Azofarbstoffe, Phthalocyanine, Isoindolinfarbstoffe und Perylentetracarbonsäurederivate besonders wirksam. Gute Ergebnisse werden mit Perylen-3,4:9,10-tetracarbonsäurediimidderivaten erzielt, wie sie in den De-OS 31 10 954 und 31 10 960 beschrieben sind.

Für die jeweilige Verwendung kann das erfindungsgemässe elektrophotographische Aufzeichnungsmaterial übliche Zusätze enthalten, z.B. Verlaufmittel und Weichmacher in der photoleitfähigen Schicht oder Haftvermittler zwischen Träger und Schicht.

Die erfindungsgemässen elektrophotographischen Aufzeichnungsmaterialien zeichnen sich durch eine Kombination sehr guter Eigenschaften, insbesondere einer hohen Photoleitfähigkeit bei gleichzeitig sehr niedriger Dunkelleitfähigkeit aus, so dass die Schichten für die Kopiertechnik sehr geeignet sind.

Deutliche Vorteile weisen sie bei der Verwendung für die Herstellung von elektrophotographischen Druckformen auf und genügen hierbei hohen Ansprüchen im Hinblick auf das Auflösungsvermögen und die Druckauflage. Die hohe Lichtempfindlichkeit erlaubt eine Senkung der Belichtungszeit bei der Verarbeitung in der Reprokamera gegenüber handelsüblichen Materialien bis etwa um die Hälfte. Aus einer sehr randscharfen Bildwiedergabe resultiert eine gute Auflösung. Durch einen hohen Ladungskontrast können auch feine Rasterpunkte in den lichten Tonwortbereichen gut wiedergegeben werden. Ferner führt die Belichtung der Schichten zu sehr geringen Restspannungen und die bei der Betonerung erhaltenen Bilder zeichnen sich durch gute Grundfreiheit in den Nichtbildbereichen aus. Die spektrale Empfindlichkeit sinkt bei 600 nm stark ab, so dass die Schichten bei Rotlicht gehandhabt werden können, ohne dass Bildverluste auftreten.

Die Herstellung elektrophotographischer Offsetdruckformen erfolgt dabei wie üblich durch eine elektrostatische Aufladung des elektrophotographischen Aufzeichnungsmaterials mittels einer Hochspannungscorona, eine direkt nachfolgende bildmässige Belichtung, die Entwicklung des vorliegenden elektrostatischen, latenten Ladungsbildes mittels eines Trocken- oder Flüssigtoners, die Fixierung des Toners durch einen nachgeschalteten Schmelzvorgang und die Entfernung der unbetonerten, photohalbleitenden Schicht mittels eines geeigneten Auswaschlösemittels. Die so erhaltene Druckform kann in bekannter Weise für den Ottsetdruck noch vorbereitet werden, z.B. durch eine Hydrophilierung und Gummierung der wasserführenden Oberfläche.

Heterocyclenderivate wie Oxdiazolderivate (vgl. DE-PS 1 058 836), Oxazolderivate (vgl. DE-PS 1 120 875) oder 2,5-Bis (4'-dialkylaminophenyl) -triazole-1,3,4 (vgl. DE-AS 10 60 260) waren zwar als Ladungsträger transportierende Verbindungen bekannt, jedoch konnte sicher die mit den erfindungsgemäss verwendeten Aminophenyl-benztriazolen erzielte Kombination vorteilhafter Eigenschaften nicht vorhergesehen werden. In der DE-OS 27 37 334 wird zwar Benztriazol zusammen mit einem Bindemittel, einer reduzierbaren Metallverbindung und einem reduzierenden Mittel für die reduzierbare Metallverbindung in einer den elektrischen Strom leitenden Masse für ein wärmeentwickelbares Bildaufzeichnungselement verwendet, doch dürfte diese Literaturstelle die vorliegende Erfindung mit ihren Ergebnissen nicht nahelegen.

Die folgenden Beispiele sollen die Erfindung zusätzlich erläutern. Die genannten Teile und Prozente beziehen sich auf das Gewicht.

Die in den Beispielen angegebenen xerographischen Messgrössen A bis G werden wie folgt ermittelt:

Die Schichten werden mit einer Gleichspannungscorona von −7,5 kV in 1 cm Abstand gleichmässig auf ein Oberflächenpotential von 600 Volt aufgeladen und dann mit dem weissen Licht einer Xenonlampe mit einer Beleuchtungsstärke von etwa 0,85 $mW \cdot cm^{-2}$ belichtet. Es werden gemessen:

Messgrösse A: Zeit in Millisekunden (ms), innerhalb der das vor Belichtung vorhandene Oberflächenpotential bei aktinischer Belichtung um die Hälfte (300 V) abgefallen ist.

Messgrösse B: Potentialabfall in der gleichen Zeit in Volt (V), der im Dunkeln infolge Dunkelleitfähigkeit der Schichten eintritt.

Messgrösse C: Nach einer Beladungszeit von 20 Sekunden erreichtes Oberflächenpotenial in Volt (V).

Messgrösse D: Potentialabfall in %, bezogen auf Messgrösse C, im Dunkeln innerhalb von 20 Sekunden.

Messgrösse E: Durch die aktinische Belichtung hervorgerufener Potentialabfall in %, bezogen auf das Ausgangspotential unmittelbar vor der Belichtung.

Messgrösse F: Potentialänderung pro Sekunde zu Beginn der Belichtung (V/s) bei einem Ausgangspotential von 1000 Volt.

Messgrösse G: Potentialdifferenz in Volt (V) zwischen belichteten und unbelichteten Flächen der beladenen Schichten.

Bei den in den Beispielen eingesetzten Benztriazolen (1) bis (15) handelt es sich um die weiter oben als sehr geeignete Verbindungen unter den Nummern (1) bis (15) angeführten und formel- und schmelzpunktmässig gekennzeichneten Benztriazole.

Beispiele 1 bis 6

Auf eine Polyethylenterephthalatfolie mit einer aufgedampften, leitfähigen Aluminiumschicht in einer Dicke von etwa 30 nm (300 Å) wird eine Schicht aus 60 Teilen eines chlorierten Perylen-3,4:9,10-tetracarbonsäurediimidbisbenzimidazols mit einem Chlorgehalt von etwa 38% und 50 Teilen eines Copolymerisats aus Vinylchlorid, Acrylsäure und einem Maleinsäurediester in einer Dicke von etwa 0,55 µm als Ladungsträger erzeugende Schicht aufgebracht.

Auf diese Ladungsträger erzeugende Schicht wird aus einer Lösung in Essigsäureethylester eine Ladungstransportschicht aus 55 Teilen eines handelsüblichen Polycarbonat-Bindemittels mit einem Schmelzbereich von 220 bis 230 °C und 40 Teilen jeweils des in der Tabelle 1 angeführten Benztriazols so aufgebracht, dass nach dem Ablüften des Lösungsmittels und 30 minütigem Trocknen bei 80 °C eine Trockenschichtdicke von 12 µm resultiert.

Tabelle 1

Xerographische Messgrössen der Aufzeichnungsmaterialien der Beispiele 1 bis 6.

| Beispiel | Benztriazol | Messgrösse A (ms) | Messgrösse B (V) | Messgrösse C (V) |
|---|---|---|---|---|
| 1 | ( 1) | 190 | 0,25 | 2 900 |
| 2 | ( 2) | 270 | 0,2 | 2 600 |
| 3 | ( 3) | 200 | 0,3 | 3 000 |
| 4 | ( 4) | 155 | 0,85 | 1 550 |
| 5 | ( 5) | 230 | 0,2 | 3 000 |
| 6 | (12) | 145 | 0,3 | 1 600 |

Wie die Messergebnisse zeigen, weisen die erfindungsgemässen elektrophotographischen Aufzeichnungsmaterialien eine hohe Photoleitfähigkeit und eine niedrige Dunkelleitfähigkeit auf. So zeigt z.B. die Schicht von Beispiel 1 im Dunkeln innerhalb von etwa 0,2 Sekunden einen Potentialabfall von 600 auf 599,8 Volt auf, während dieselbe Schicht in der gleichen Zeit bei Belichtung mit einer Beleuchtungsstärke von 0,85 $mW \cdot cm^{-2}$ von 600 auf 300 Volt abfällt. Die maximale Beladbarkeit ist mit grösser 1 500 Volt weit über den in Kopiergeräten erforderlichen Oberflächenpotentialen (ca. 700 Volt), so dass die Schichten für die Kopiertechnik sehr geeignet sind.

Beispiel 7

Es wird wie in den Beispielen 1 bis 6 verfahren, jedoch wird die Ladungstransportschicht aus 55 Teilen eines Copolymerisats aus 80% Styrol und 20% Maleinsäureanhydrid und aus 45 Teilen 2-(4'-Ethylbenzylaminophenyl)-benztriazol -1,2,3 hergestellt. Das Aufzeichnungsmaterial zeigt bei den xerographischen Messungen im Dunkeln innerhalb von 20 Sekunden einen Potentialabfall um 15%, bei Belichtung mit weissem Licht einer Xenonhochdrucklampe von 0,85 $mW \cdot cm^{-2}$ Beleuchtungsstärke auf der Schicht einen Potentialabfall um die Hälfte in 0,22 Sekunden. Wird diese Schicht als Kopierfolie in einem handelsüblichen Kopiergerät mit Trockentoner verwendet, so können damit Kopien von guter Qualität und in hoher Anzahl hergestellt werden.

Beispiele 8 bis 22

55 Teile eines Copolymerisats aus 70% Styrol, 6% Maleinsäureanhydrid und 24% Acrylsäure mit einem mittleren Molekulargewicht von ca. 2000, 45 Teile des jeweiligen, in der Tabelle 2 angegebenen Benztriazols (1) bis (15) und 0,3 Teile Methylviolett (C.I. 42535) werden in Essigsäureethylester gelöst, und die Lösung wird auf einen elektrisch leitfähigen Träger aus einem elektrolytisch aufgerauhten und danach anodisch oxidierten Aluminiumblech von 0,15 mm Dicke so aufgetragen, dass nach dem Ablüften des Lösungsmittels und 30 minütigem Trocknen bei 85 °C eine Trockenschicht von 4 µm resultiert. Die xerographischen Messgrössen zeigt Tabelle 2.

Tabelle 2

Xerographische Messgrössen der Aufzeichnungsmaterialien der Beispiele 8 bis 22.

| Beispiel | Benztriazol | Messgrösse A (ms) | C (Volt) | D (%) | E (%) | F (Volt/s) | G (Volt) |
|---|---|---|---|---|---|---|---|
| 8 | ( 1) | 190 | 600 | 14 | 93 | 2540 | 470 |
| 9 | ( 2) | 215 | 520 | 25 | 95 | 2000 | 370 |
| 10 | ( 3) | 185 | 540 | 16 | 91 | 2420 | 410 |
| 11 | ( 4) | 210 | 580 | 17 | 91 | 2120 | 440 |
| 12 | ( 5) | 190 | 600 | 19 | 96 | 2360 | 470 |
| 13 | ( 6) | 270 | 670 | 31 | 82 | 1870 | 460 |
| 14 | ( 7) | 160 | 660 | 18 | 95 | 2960 | 540 |
| 15 | ( 8) | 240 | 680 | 33 | 81 | 1800 | 460 |
| 16 | ( 9) | 260 | 510 | 25 | 78 | 1825 | 380 |
| 17 | (10) | 190 | 770 | 31 | 88 | 1980 | 530 |
| 18 | (11) | 210 | 540 | 25 | 82 | 1800 | 410 |
| 19 | (12) | 160 | 570 | 19 | 95 | 2700 | 460 |
| 20 | (13) | 170 | 680 | 16 | 91 | 2750 | 570 |
| 21 | (14) | 190 | 660 | 25 | 89 | 2580 | 500 |
| 22 | (15) | 240 | 580 | 20 | 86 | 1875 | 470 |

Beispiel 23

50 Teile eines Copolymerisats aus 60% Styrol und 40% einer mit Methanol halbveresterten Maleinsäure mit einem mittleren Molekulargewicht $\overline{M}_w$ von 10 000, 50 Teile 2-(4'-Diethylaminophenyl)-benztriazol-1,2,3 und 0,2 Teile Kristallviolett (C.I. 42555) werden aus einer 5%igen Lösung in Tetrahydrofuran auf eine elektrolytisch aufgerauhte und anodisierte Aluminiumfolie von 0,15 mm Dicke in einer Trockenschichtdicke von etwa 4 µm aufgebracht.

Diese Druckplatte wird nach einer Aufladung mittels einer Hochspannungscorona in einer Kamera bildmässig 25 Sekunden belichtet. Danach wird mit einem Pulvertoner entwickelt, der bei 160 °C abriebfest eingebrannt wird. Die unbetonerte Schicht wird mit einem Gemisch aus 0,5% Soda, 25% Isopropanol und 74,5% Wasser abgewaschen, wodurch die Aluminiumoberfläche freigelegt wird. Die Lösungen werden mit einem Wattebausch über die Schicht gestrichen. Man erhält die im Offsetdruck erwünschte Differenzierung in hydrophile und oleophile Bereiche, wobei die Trägeroberfläche die hydrophilen Bereiche liefert.

Anschliessend an die Behandlung mit der alkalischen Flüssigkeit wird die Druckplatte mit Wasser nachgespült und durch Überwischen mit verdünnter Phosphorsäurelösung die Hydrophilie der Trägeroberfläche weiter erhöht. Nach Einfärben mit fetter Farbe wird auf bekannte Weise in Offsetdruckmaschinen damit gedruckt.

## Patentansprüche

1. Elektrophotographische Aufzeichnungsmaterialien mit elektrisch leitenden Trägern, Ladungsträger erzeugenden Verbindungen und Ladungsträger transportierenden Verbindungen, dadurch gekennzeichnet, dass sie als Ladungsträger transportierende Verbindungen solche der Formel (I) enthalten

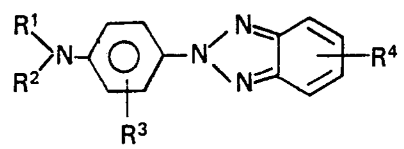 (I)

worin bedeuten:

$R^1$ und $R^2$ = Wasserstoff, Alkyl, Allyl, Benzyl oder ggf. subst. Phenyl, gleich oder verschieden, oder $R^1$ und $R^2$ gemeinsam

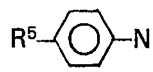 =oder 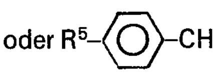 =mit $R^5$ = $NR^1R^2$

$R^3$ = Wasserstoff, Alkyl, Alkoxy oder Halogen,

$R^4$ = Wasserstoff, Alkyl, Alkoxy, Vinyl, Allyl, Dialkylamino, Nitro, Cyan oder Acryloyl.

2. Elektrophotographische Aufzeichnungsmaterialien gemäss Anspruch 1, dadurch gekennzeichnet, dass in Formel (I) bedeuten

$R^1$ = Wasserstoff, Alkyl, Benzyl oder Phenyl

$R^2$ = Alkyl, Benzyl oder Phenyl

$R^3$ = Wasserstoff und

$R^4$ = Wasserstoff, Alkoxy oder Halogen.

3. Elektrophotographische Aufzeichnungsmaterialien gemäss Anspruch 1, dadurch gekennzeichnet, dass sie einen elektrisch leitenden Träger, eine Schicht mit Ladungsträger erzeugenden Verbindungen und eine weitere Schicht mit Ladungsträger transportierenden Verbindungen gemäss der in Anspruch 1 gegebenen Formel (I) umfassen.

4. Aufzeichnungsmaterialien zur Herstellung elektrophotographischer Druckformen, insbesondere Offsetdruckformen, gemäss Ansprüchen 1 oder 2, dadurch gekennzeichnet, dass sie auf einem für Druckformen geeigneten Trägermaterial einer Dicke von 0,08 bis 0,6 mm eine photohalbleitende Schicht aus

a) mindestens einem Bindemittel,

b) mindestens einer Ladungsträger transportierenden Verbindung gemäss Ansprüchen 1 oder 2

c) mindestens einem Farbstoff als Sensibilisator und

d) ggf. weiteren Zusätzen aufweist.

5. Aufzeichnungsmaterialien gemäss Anspruch 4, dadurch gekennzeichnet, dass das Bindemittel in basischen wässrigen oder alkoholischen Lösungsmitteln löslich ist.

6. Aufzeichnungsmaterialien gemäss Asnpruch 5, dadurch gekennzeichnet, dass das Bindemittel ein mittleres Molekulargewicht von 800 bis 50 000 aufweist.

7. Aufzeichnungsmaterialien gemäss Ansprüchen 5 oder 6, dadurch gekennzeichnet, dass das Bindemittel ein Styrol-Maleinsäureanhydrid-Acryl- oder Methacrylsäure-Copolymerisat mit einem Anteil an einpolymerisierten Maleinsäureanhydrid-Gruppen von 5 bis 50 Gew.-% und einem Anteil an einpolymerisierten Acryl- und/oder Methacrylsäure-Gruppen von 5 bis 35 Gew.-% ist.

8. Aufzeichnungsmaterial gemäss einem der Ansprüche 1 bis 7, dadurch gekennzeichnet, dass die Bestandteile der elektrophotographischen Schicht in Form von Lösungen auf den Schichtträger aufgetragen wurden.

9. Verwendung der elektrophotographischen Aufzeichnungsmaterialien gemäss Ansprüchen 1 bis 3 für reprographische Zwecke.

10. Verwendung der elektrophotographischen Aufzeichnungsmaterialien gemäss Ansprüchen 1, 2 und 4 bis 8 zur Herstellung elektrophotographischer Druckformen, insbesondere Offsetdruckformen.

## Revendications

1. Matériaux d'enregistrement électrophotographique munis de supports conducteurs de l'électricité, de composés générant des porteurs de charge et de composés transportant des porteurs de charge, caractérisés par le fait qu'ils contiennent, comme composés transportant des porteurs de charge, ceux de formule (I)

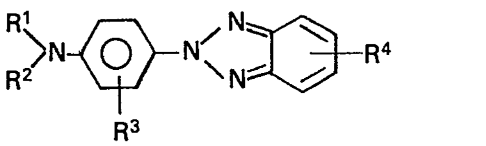

(I)

où

$R^1$ et $R^2$ représentent, identiques ou différents, hydrogène, alkyle, allyle, benzyle ou phényle éventuellement substitué, ou $R^1$ et $R^2$ ensemble

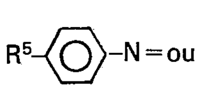 ou 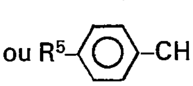 avec $R^5 = NR^1R^2$

$R^3$ = hydrogène, alkyle, alkoxy ou halogène

$R^4$ = hydrogène, alkyle, alcoxy, vinyle, allyle, dialkylamino, nitro, cyano ou acryloyle.

2. Matériaux d'enregistrement électrophotographique selon la revendication 1, caractérisés par le fait que dans la formule (I)

$R^1$ = hydrogène, alkyle, benzyle ou phényle

$R^2$ = alkyle, benzyle ou phényle

$R^3$ = hydrogène et

$R^4$ = hydrogène, alcoxy ou halogène.

3. Matériaux d'enregistrement électrophotographique selon la revendication 1, caractérisés par le fait qu'elles comprennent un support conducteur de l'électricité, une couche à composés générant des porteurs de charge et une autre couche à composés transportant des porteurs de charge, selon la formule (I) donnée dans la revendication 1.

4. Matériaux d'enregistement pour la production de formes d'impression, en particulier de formes d'impression offset, selon les revendications 1 ou 2, caractérisés par le fait qu'ils comportent, sur un matériau support approprié aux formes d'impression, d'une épaisseur de 0,08 à 0,6 mm, une couche photo semi-conductrice de

a) au moins un liant

b) au moins un composé transportant des porteurs de charge selon les revendications 1 ou 2

c) au moins un colorant comme sensibilisateur et

d) éventuellement d'autres additifs.

5. Matériaux d'enregistrement selon la revendication 4, caractérisés par le fait que le liant est soluble dans des solvants basiques aqueux ou alcooliques.

6. Matériaux d'enregistrement selon la revendication 5, caractérisés par le fait que le liant possède un poids moléculaire moyen de 800 à 50 000.

7. Matériaux d'enregistrement selon les revendications 5 ou 6, caractérisés par le fait que le liant est un copolymérisat de styrène-anhydride maléique-acide acrylique ou méthacrylique, avec une proportion des groupes anhydride maléique polymérisés de 5 à 50% en poids et une proportion en groupes acide acrylique ou méthacrylique polymérisés de 5 à 35% en poids.

8. Matériaux d'enregistrement selon l'une des revendications 1 à 7, caractérisés par le fait que les parties constitutives de la couche électrophotographique sont appliquées sur la support de couche sous forme de solutions.

9. Utilisation des matériaux d'enregistrement électrophotographique selon les revendications 1 à 3 pour les applications reprographiques.

10. Utilisation des matéraux d'enregistrement électrophotographique selon les revendications 1, 2 et 4 à 8 pour la préparation de formes d'impression électrophotographique, en particulier de formes d'impression offset.

## Claims

1. An electrophotographic recording material comprising an electrically conductive base, charge carrier-producing compounds and charge carrier-transporting compounds, which contains, as charge carrier-transporting compounds, those of the formula (I)

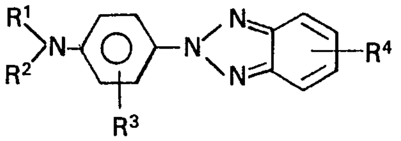

(I)

where

$R^1$ and $R^2$ are identical or different and are each hydrogen, alkyl, allyl, benzyl or unsubstituted or substituted phenyl, or

$R^1$ and $R^2$ together are 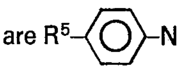 =or

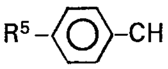 =, where $R^5$ is $NR^1R^2$,

$R^3$ is hydrogen, alkyl, alkoxy or halogen, and
$R^4$ is hydrogen, alkyl, alkoxy, vinyl, allyl, dialkylamino, nitro, cyano or acryloyl.

2. An electrophotographic recording material as claimed in claim 1, wherein, in formula (I),
$R^1$ is hydrogen, alkyl, benzyl or phenyl,
$R^2$ is alkyl, benzyl or phenyl,
$R^3$ is hydrogen, and
$R^4$ is hydrogen, alkoxy or halogen.

3. An electrophotographic recording material as claimed in claim 1, which comprises an electrically conductive base, a layer containing charge carrier-producing compounds and another layer containing charge carrier-transporting compounds of the formula (I) given in claim 1.

4. A recording material for the production of an electrophotographic printing plate, in particular an offset printing plate, as claimed in claim 1 or 2, which comprises, on a 0.08–0.6 mm thick base suitable for printing plates, a photosemiconducting layer containing
a) one or more binders,
b) one or more charge carrier-transporting compounds as claimed in claim 1 or 2 and
c) one or more dyes as sensitizers, with or without
d) further additives.

5. A recording material as claimed in claim 4, wherein the binder is soluble in basic aqueous or alcoholic solvents.

6. A Recording material as claimed in claim 5, wherein the binder has a mean molcular weight of from 800 to 50 000.

7. A recording material as claimed in claim 5 or 6, wherein the binder is a styrene/maleic anhydride/acrylic or methacrylic acid copolymer containing from 5 to 50% by weight of maleic anhydride as copolymerized units, and from 5 to 35% by weight of acrylic and/or methacrylic acid as copolymerized units.

8. A recording material as claimed in any of claims 1 to 7, wherein the components of the electrophotographic layer are applied to the base in the form of a solution.

9. The use of an electrophotographic recording material as claimed in claims 1 to 3 for reprographic purposes.

10. The use of an electrophotographic recording material as claimed in claims 1, 2 and 4 to 8 for the production of electrophotographic printing plates, in particular offset printing plates.